# EUROPEAN PATENT APPLICATION

(11) **EP 1 681 284 A1**
(43) Date of publication of application: **19.07.2006**
(21) Application number: 04792998.9
(22) Date of filing: 20.10.2004
(51) Int. Cl.: C07C 251/08, C07C 251/18, C07C 251/20, C08G 73/06

(54) **AROMATIC POLYAMINE DERIVATIVE**

(30) Priority: 05.11.2003 JP 2003376049; 13.11.2003 JP 2003383223
(71) Applicant: DAICEL CHEMICAL INDUSTRIES, LTD., Sakai-shi, Osaka 590-8501 (JP)
(72) Inventor: NAGANO, Shinya, Himeji-shi, Hyogo 6711204 (JP); HASHIMOTO, Jiichiro, H imeji-shi, Hyogo 6711262 (JP)
(74) Representative: Grünecker, Kinkeldey, Stockmair & Schwanhäusser Anwaltssozietät
(86) International application number: PCT/JP2004/015882
(87) International publication number: WO 2005/044781

(57) **Abstract**

Aromatic polyamine derivatives of this invention are represented by following Formula (1): 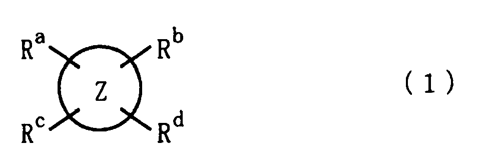 wherein Ring Z represents a monocyclic or polycyclic aromatic ring; R^{a}, R^{b}, R^{c}, and R^{d} are each a substituent bonded to Ring Z, where R^{a} and R^{b} are the same as or different from each other and each represent a protected or unprotected amino group, and R^{c} and R^{d} are the same as or different from each other and each represent a protected or unprotected amino group, a protected or unprotected hydroxy group, or a protected or unprotected mercapto group, and wherein at least one of R^{a}, R^{b}, R^{c}, and R^{d} represents an amino group protected by an alkylidene group.

## Description

### Technical Field

The present invention relates to novel aromatic polyamine derivatives useful for forming films of polyazoles, such as imidazoles, oxazoles, and thiazoles, which have high heat resistance and low dielectric constants.

### Background Art

Polybenzazoles having adamantane skeletons are useful as highly thermostable resins (see, for example, "Journal of Polymer Science" Part A-1 (1970), 8(12), p.3665-6). Among them, highly crosslinked polybenzazoles using trifunctional or tetrafunctional adamantanes are very useful as materials for interlayer dielectric films, since they have a multiplicity of molecular-scale pores and thereby show low relative dielectric constants and sufficient mechanical strength and heat resistance (see, for example, Japanese Unexamined Patent Application Publication (JP-A) No. 2001-332543). These highly crosslinked polybenzazoles can be synthetically prepared, for example, by a production process such as heating in the presence of a condensing agent such as a polyphosphoric acid. The resulting highly crosslinked resins, however, are very insoluble in a solvent, cannot significantly be applied as a thin film to a substrate typically by coating, and films having thicknesses necessary as interlayer dielectric films cannot be significantly formed.

A thin film of a wholly aromatic chain polybenzazole is formed by a certain method including the steps of spreading an aldehyde derivative as a material monomer over an aqueous solution containing an amine monomer as another material monomer; allowing polymerization to proceed to thereby form a film on a gas-liquid interface; laminating formed films on a substrate by a horizontal attachment method; and carrying out heat treatment in the air (see, for example, Japanese Unexamined Patent Application Publication (JP-A) No. 62-183881). However, this method is not suitable for industrial production, since it takes quite a long time to form the target thin film. In addition, the precursor polyimine is subjected to an oxidative thermal treatment in a final process, and the resulting polybenzazole film may be possibly oxidized, thus a lower dielectric constant necessary as a dielectric film is not expected.

Additionally, aromatic polyamines used as the material monomer have a considerably high polarity as compounds and are soluble in quite limited types of solvents. Particularly, these monomers are hardly soluble in a solvent having a low polarity, and a film having a thickness of several hundred nanometers required as an interlayer dielectric films for semiconductors cannot significantly be formed. For such reasons, it has been difficult to form dielectric films of highly crosslinked polybenzazoles using aromatic polyamines.

### Disclosure of Invention

An object of the present invention is to provide a novel aromatic polyamine derivative that can constitute a highly crosslinked polybenzazole and is useful as a material for forming dielectric films that can easily have a film thickness necessary as interlayer dielectric films.

Another object of the present invention is to provide a novel aromatic polyamine derivative that is useful for forming dielectric films of polybenzazoles which show high heat resistance and low dielectric constants and are useful typically for semiconductor components.

After intensive investigations to achieve the above objects, the present inventors have found that an aromatic polyamine derivative with at least one of four amino groups being protected by a specific protecting group has a markedly increased solubility in a solvent, and that a material for forming dielectric films which has a high monomer concentration can be obtained by using this derivative as a monomer component, and that this material can form a dielectric film having a sufficient film thickness necessary typically as interlayer dielectric films. The present invention has been achieved based on these findings.

Specifically, the present invention provides an aromatic polyamine derivative represented by following Formula (1): 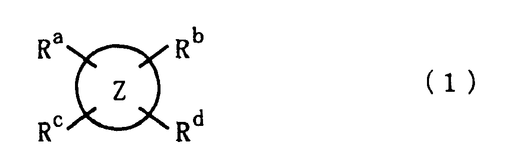 wherein Ring Z represents a monocyclic or polycyclic aromatic ring; R^{a}, R^{b}, R^{c}, and R^{d} are each a substituent bound to Ring Z, wherein R^{a}, R^{b} are the same as or different from each other and each represent a protected or unprotected amino group, and R^{c} and R^{d} are the same as or different from each other and each represent a protected or unprotected amino group, a protected or unprotected hydroxy group, or a protected or unprotected mercapto group, and wherein at least one of R^{a}, R^{b}, R^{c}, and R^{d} represents an amino group protected by an alkylidene group.

The present invention provides novel aromatic polyamine derivatives. These aromatic polyamine derivatives have markedly improved solubilities in a solvent, and materials for forming dielectric films using these aromatic polyamine derivatives can form dielectric films that have a sufficient film thickness and contain a highly crosslinked polybenzazole containing an adamantane skeleton. The materials can have increased solubilities in various solvents to thereby yield polybenzazole films having thickness within a broad range corresponding to a variety of semiconductor fabrication processes. The resulting dielectric films formed from the materials can exhibit high heat resistance and low dielectric constants.

### Brief Description of the Drawings

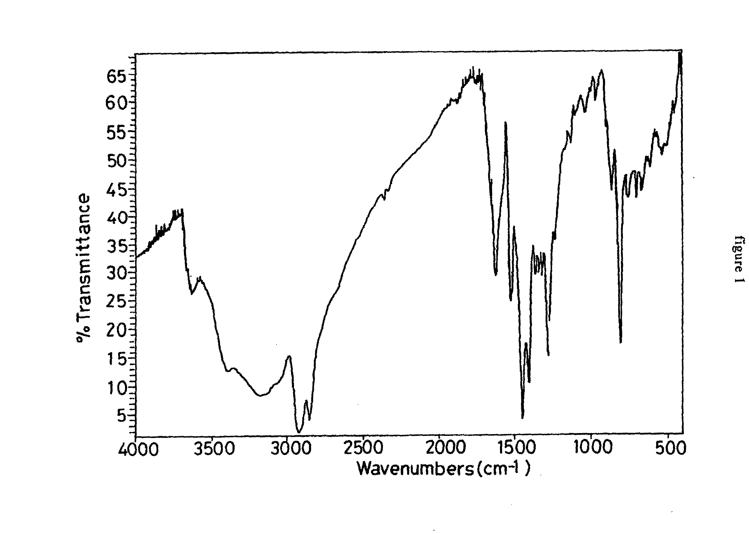

FIG. 1 is an infrared absorption spectrum of a polymer film obtained according to Example 15.

### Best Mode for Carrying Out the Invention

The aromatic polyamine derivatives according to the present invention are represented by Formula (1). In Formula (1), the aromatic ring in Ring Z includes monocyclic or polycyclic aromatic hydrocarbon rings and heteroaromatic rings. The monocyclic aromatic hydrocarbon rings include benzene ring. Examples of the polycyclic aromatic hydrocarbon rings include those having a fused ring structure, in which two or more aromatic rings each possess two or more two or more atoms in common, such as naphthalene ring, anthracene ring, phenanthrene ring, and phenalene ring; and those having a structure in which two or more aromatic rings bonded with the interposition of a linkage group such as a single bond, or an alicyclic ring, such as biphenyl ring, biphenylene ring, and fluorene ring. The heteroaromatic rings include monocyclic or polycyclic heteroaromatic rings each containing one or more hetero atoms such as oxygen atoms, sulfur atoms, and nitrogen atoms. Concrete examples of the heteroaromatic rings include monocyclic rings such as furan ring, thiophene ring, pyridine ring, and picoline ring; and polycyclic rings such as quinoline ring, isoquinoline ring, acridine ring, and phenazine ring. The aromatic ring may have one or more substituents. These substituents are not specifically limited, as long as they do not adversely affect the reaction. Representative examples of the substituents include halogen atoms such as bromine, chlorine, and fluorine atoms; aliphatic hydrocarbon groups including alkyl groups having one to four carbon atoms, such as methyl, ethyl, propyl, butyl, and t-butyl; alicyclic hydrocarbon groups including cycloalkyl groups having about three to about fifteen members, such as cyclohexyl group; aromatic hydrocarbon groups including aromatic hydrocarbon groups having about six to about twenty carbon atoms, and preferably having about six to about fourteen carbon atoms, such as phenyl, benzyl, naphthyl, and toluyl groups.

Examples of the protecting groups for the amino group in R^{a} and R^{b} in Formula (1) include acyl groups including aliphatic acyl groups having one to six carbon atoms, such as formyl, acetyl, propionyl, butyryl, isobutyryl, valeryl, and pivaloyl groups, and aromatic acyl groups having about six to about twenty carbon atoms, such as benzoyl, and naphthoyl groups; alkoxycarbonyl groups including (C₁₋₄ alkoxy)-carbonyl groups such as methoxycarbonyl, ethoxycarbonyl, and t-butoxycarbonyl; aralkyloxy-carbonyl groups including (C₇₋₂₀ aralkyl)oxycarbonyl groups such as benzyloxycarbonyl group, and p-methoxybenzyloxycarbonyl group; alkylidene groups including aliphatic alkylidene groups such as methylidene, ethylidene, propylidene, isopropylidene, butylidene, isobutylidene, pentylidene, cyclopentylidene, hexylidene, cyclohexylidene, and cyclohexanomethylidene groups, and aromatic alkylidene groups such as benzylidene and methylphenylmethylidene.

The protected amino groups further include mono-substituted amino groups within ranges not adversely affecting the reaction for conversion into polybenzazoles. Examples of the mono-substituted amino groups include alkylamino groups such as methylamino group, ethylamino group, propylamino group, butylamino group, and t-butylamino group; cycloalkylamino groups such as cyclohexylamino group; arylamino groups such as phenylamino group; and aralkylamino groups such as benzylamino group. The amino-protecting groups are not limited to these, and any groups that are conventionally used in the field of organic synthesis can be used.

The amino-protecting groups in R^{c} and R^{d} include those listed as the amino-protecting groups in R^{a} and R^{b}. The amino-protecting-groups also include protecting groups capable of protecting plural amino groups simultaneously (polyfunctional protecting groups). Examples of such protecting groups include carbonyl group, oxalyl group, and butane-2,3-diylidene group. When a protecting group of this type is used, R^{a} and R^{b} (R^{c} and R^{d}, R^{a} and R^{c}, or R^{b} and R^{d}) are simultaneously protected by one polyfunctional protecting group, to thereby form a ring adjacent to Ring Z. Examples of the hydroxy-protecting groups include alkyl groups including alkyl groups having one to six carbon atoms, such as methyl, ethyl, propyl, isopropyl, butyl, t-butyl, pentyl, and hexyl groups; cycloalkyl groups including cycloalkyl groups having three to fifteen members, such as cyclopentyl group and cyclohexyl group; aralkyl groups including aralkyl groups having seven to twenty carbon atoms, such as benzyl group; substituted methyl groups including substituted methyl groups having, in total, about two to about ten carbon atoms, such as methoxymethyl, benzyloxymethyl, t-butoxymethyl, and 2-methoxyethoxymethyl groups; substituted ethyl groups such as 1-ethoxyethyl, and 1-methyl-1-methoxyethyl groups; acyl groups including aliphatic acyl groups having one to ten carbon atoms, such as formyl, acetyl, propionyl, butyryl, isobutyryl, valeryl, and pivaloyl groups, alicyclic acyl groups having four to twenty carbon atoms, such as cyclohexylcarbonyl group, and aromatic acyl groups having seven to twenty carbon atoms, such as benzoyl, and naphthoyl groups; alkoxycarbonyl groups including (C₁₋₄ alkoxy)-carbonyl groups such as methoxycarbonyl, ethoxycarbonyl, and t-butoxycarbonyl; and aralkyloxycarbonyl groups including (C₇₋₂₀ aralkyl)oxycarbonyl groups such as benzyloxycarbonyl group and p-methoxybenzyloxycarbonyl group. The mercapto-protecting groups can be groups listed as the hydroxy-protecting groups.

R^{c} and R^{d} in Ring Z of Formula (1) are preferably positioned, for example, at the alpha-position or beta-position, respectively, with respect to the carbon atoms having R^{a} and R^{b} as protected or unprotected amino groups in Ring Z.

For example, by allowing an aromatic polyamine derivative having R^{c} (R^{d}) at the alpha-position with respect to the carbon atom having R^{a} (R^{b}) in Ring Z of Formula (1) to react with an adamantanepolycarboxylic acid or a derivative thereof, the protecting group of amino group and/or carboxyl group is generally eliminated, to thereby form a five-membered azole ring. Specifically, an imidazole ring, an oxazole ring, or an thiazole ring, for example, is formed when R^{c} is a protected or unprotected amino group, a protected or unprotected hydroxy group, or a protected or unprotected mercapto group, respectively.

By allowing an aromatic polyamine derivative having R^{c} (R^{d}) at the beta-position with respect to the carbon atom having R^{a} (R^{b}) in Ring Z of Formula (1) to react with an adamantanepolycarboxylic acid or a derivative thereof, the protecting group of amino group and/or carboxyl group is generally eliminated, to thereby form a six-membered nitrogen-containing ring. Specifically, a hydrodiazine ring, an oxazine ring, or a thiazine ring, for example, is formed when R^{c} is an amino group or mono-substituted amino group, a hydroxy group, or a mercapto group, respectively.

In Formula (1), the positions of R^{a} and R^{b} in Ring Z are not specifically limited, as long as these groups can be combined with a carboxyl group in an adamantanepolycarboxylic acid to form, for example, a five-or six-membered ring together with the adjacent carbon atom. The positions are preferably such that R^{a} and R^{b} [two -NH₂S] are not adjacent to each other.

The aromatic polyamine derivatives according to the present invention are compounds of Formula (1) in which at least one of R^{a}, R^{b}, R^{c}, and R^{d} is an amino group protected by an alkylidene group. Namely, they are imine derivatives.

Representative examples of the aromatic polyamine derivatives according to the present invention are shown below, by taking compounds having a benzene ring or biphenyl ring as the aromatic Ring Z and each having two or four groups protected by a protecting group as an example. The aromatic polyamine derivatives according to the present invention are not limited to these compounds.

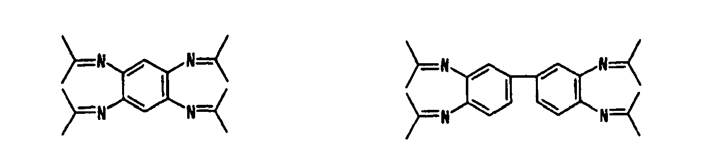

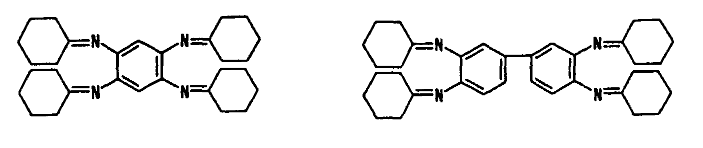

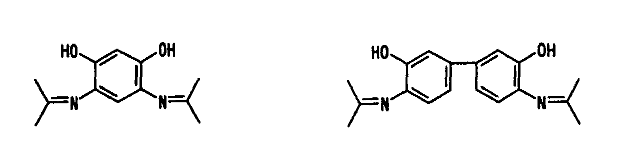

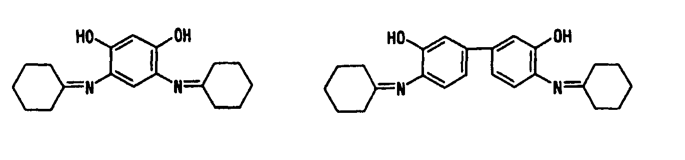

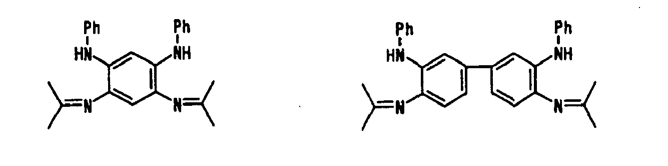

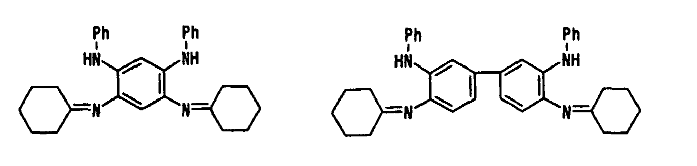

Examples of the aromatic polyamine derivatives according to the present invention include imine derivatives of Formula (1), in which Z is a benzene ring; and R^{a} and R^{b} are each an amino group protected by an alkylidene group, including those in which R^{c} and R^{d} are each an amino group, such as N,N"-diisopropylidene-1,2,4,5-benzenetetramine, N,N'"-diisopropylidene-1,2,4,5-benzenetetramine, N,N"-dicyclohexylidene-1,2,4,5-benzenetetramine, N,N'"-dicyclohexylidene-1,2,4,5-benzenetetramine, N,N"-dibenzylidene-1,2,4,5-benzenetetramine, and N,N'"-dibenzylidene-1,2,4,5-benzenetetramine; and those in which R^{c} and R^{d} are each an amino group protected by an alkylidene group, such as N,N',N",N'"-tetraisopropylidene-1,2,4,5-benzenetetramine, N,N',N",N'"-tetracyclohexylidene-1,2,4,5-benzenetetramine, and N, N', N", N'"-tetrabenzylidene-1,2,4,5-benzenetetramine.

The aromatic polyamine derivatives according to the present invention further include, for example, imine derivatives of Formula (1), in which Z is a benzene ring; and R^{a} and R^{b} are each an amino group protected by an alkylidene group, including those in which R^{c} and R^{d} are each a mono-substituted amino group, such as N,N"-diisopropylidene-N',N'"-dimethyl-1,2,4,5-benzenetetramine, N,N'"-diisopropylidene-N',N"-dimethyl-1,2,4,5-benzenetetramine, N,N"-dicyclohexylidene-N',N"'-dimethyl-1,2,4,5-benzenetetramine, N,N'"-dicyclohexylidene-N',N'"-dimethyl-1,2,4,5-benzenetetramine, N,N"-dibenzylidene-N',N'"-dimethyl-1,2,4,5-benzenetetramine, N,N'"-dibenzylidene-N',N'"-dimethyl-1,2,4,5-benzenetetramine, N,N" -diisopropylidene-N',N'"-diphenyl-1,2,4,5-benzenetetramine, N,N"'-diisopropylidene-N',N"-diphenyl-1,2,4,5-benzenetetramine, N,N" -dicyclohexylidene-N',N"'-diphenyl-1,2,4,5-benzenetetramine, N,N'"-dicyclohexylidene-N',N"'-diphenyl-1,2,4,5-benzenetetramine, N,N"-dibenzylidene-N',N"'-diphenyl-1,2,4,5-benzenetetramine, and N,N"'-dibenzylidene-N',N"'-diphenyl-1,2,4,5-benzenetetramine.

In addition to the above compounds, the aromatic polyamine derivatives according to the present invention further include imine derivatives in which R^{c} and R^{d} are each a hydroxy group, such as N,N'-diisopropylidene-2,5-dihydroxy-1,4-benzenediamine, N,N'-diisopropylidene-2,4-dihydroxy-1,5-benzenediamine, N,N'-dicyclohexylidene-2,5-dihydroxy-1,4-benzenediamine, N,N'-dicyclohexylidene-2,4-dihydroxy-1,5-benzenediamine, N,N'-dibenzylidene-2,5-dihydroxy-1,4-benzenediamine, and N,N'-dibenzylidene-2,4-dihydroxy-1,5-benzenediamine; and imine derivatives in which R^{c} and R^{d} are each a mercapto group, such as N,N'-diisopropylidene-2,5-dimercapto-1,4-benzenediamine, N,N'-diisopropylidene-2,4-dimercapto-1,5-benzenediamine, N,N'-dicyclohexylidene-2,5-dimercapto-1,4-benzenediamine, N,N'-dicyclohexylidene-2,4-dimercapto-1,5-benzenediamine, N,N'-dibenzylidene-2,5-dimercapto-1,4-benzenediamine, and N,N'-dibenzylidene-2,4-dimercapto-1,5-benzenediamine.

The aromatic polyamine derivatives according to the present invention further include, for example, imine derivatives of Formula (1) in which Z is a biphenyl ring; and R^{a} and R^{b} are each an amino group protected by an alkylidene group, including those in which R^{c} and R^{d} are each an amino group, such as N,N " -diisopropylidene-3,4,3',4'-biphenyltetramine, N,N"'-diisopropylidene-3,4,3',4'-biphenyltetramine, N,N"-diisobutylidene-3,4,3',4'-biphenyltetramine, N,N"'-diisobutylidene-3,4,3',4'-biphenyltetramine, N,N"-dicyclohexylidene-3,4,3',4'-biphenyltetramine, N,N"'-dicyclohexylidene-3,4,3',4'-biphenyltetramine, N,N" -dicyclohexanomethylidene-3,4,3',4'-biphenyltetramine, N,N"'-dicyclohexanomethylidene-3,4,3',4'-biphenyltetramine, N,N" -dibenzylidene-3,4,3',4'-biphenyltetramine, and N,N"'-dibenzylidene-3,4,3',4'-biphenyltetramine, and those in which R^{c} and R^{d} are each an amino group protected by an alkylidene group, such as N,N',N",N'"-tetraisopropylidene-3,4,3',4'-biphenyltetramine, N,N',N",N'"-tetraisobutylidene-3,4,3',4'-biphenyltetramine, N,N',N",N"'-tetracyclohexylidene-3,4,3',4'-biphenyltetramine, N,N',N",N"'-tetracyclohexanomethylidene-3,4,3',4'-biphenyltetramine, and N,N',N",N"'-tetrabenzylidene-3,4,3',4'-biphenyltetramine.

Examples of the aromatic polyamine derivatives according to the present invention further include imine derivatives of Formula (1) in which Z is a biphenyl ring; and R^{a} and R^{b} are each an amino group protected by an alkylidene group, including those in which R^{c} and R^{d} are each a mono-substituted amino group, such as N,N"-diisopropylidene-N',N"'-dimethyl-3,4,3',4'-biphenyltetramine, N,N"'-diisopropylidene-N',N"-dimethyl-3,4,3',4'-biphenyltetramine, N,N"-diisobutylidene-N',N'"-dimethyl-3,4,3',4'-biphenyltetramine, N,N'"-diisobutylidene-N',N" -dimethyl-3,4,3',4'-biphenyltetramine, N,N" -dicyclohexylidene-N',N"'-dimethyl-3,4,3',4'-biphenyltetramine, N,N'"-dicyclohexylidene-N',N"'-dimethyl-3,4,3',4'-biphenyltetramine, N,N"-dicyclohexanomethylidene-N',N"'-dimethyl-3,4,3',4'-biphenyltetramine, N,N'"-dicyclohexanomethylidene-N',N'"-dimethyl-3,4,3',4'-biphenyltetramine, N,N"-dibenzylidene-N',N'"-dimethyl-3,4,3',4'-biphenyltetramine, N,N'"-dibenzylidene-N',N'"-dimethyl-3,4,3',4'-biphenyltetramine, N,N"-diisopropylidene-N',N'"-diphenyl-3,4,3',4'-biphenyltetramine, N,N'"-diisopropylidene-N',N"-diphenyl-3,4,3',4'-biphenyltetramine, N,N"-dicyclohexylidene-N',N'"-diphenyl-3,4,3',4'-biphenyltetramine, N,N'"-dicyclohexylidene-N',N'"-diphenyl-3,4,3',4'-biphenyltetramine, N,N"-dibenzylidene-N',N"'-diphenyl-3,4,3',4'-biphenyltetramine, and N,N'"-dibenzylidene-N',N' ' '-diphenyl-3,4,3',4'-biphenyltetramine.

The aromatic polyamine derivatives according to the present invention further include, in addition to the above compounds, imine derivatives in which R^{c} and R^{d} are each a hydroxy group, such as N,N'-diisopropylidene-3,3'-dihydroxy-4,4'-biphenyldiamine, N,N'-diisopropylidene-3,4'-dihydroxy-4,3'-biphenyldiamine, N,N'-diisobutylidene-3,3'-dihydroxy-4,4'-biphenyldiamine, N,N'-diisobutylidene-3,4'-dihydroxy-4,3'-biphenyldiamine, N,N'-dicyclohexylidene-3,3'-dihydroxy-4,4'-biphenyldiamine, N,N'-dicyclohexylidene-3,4'-dihydroxy-4,3'-biphenyldiamine, N,N'-dicyclohexanomethylidene-3,3'-dihydroxy-4,4'-biphenyldiamine, N,N'-dicyclohexanomethylidene-3,4'-dihydroxy-4,3'-biphenyldiamine, N,N'-dibenzylidene-3,3'-dihydroxy-4,4'-biphenyldiamine, and N,N'-dibenzylidene-3,4'-dihydroxy-4,3'-biphenyldiamine; and imine derivatives in which R^{c} and R^{d} are each a mercapto group, such as N,N'-diisopropylidene-3,3'-dimercapto-4,4'-biphenyldiamine, N,N'-diisopropylidene-3,4'-dimercapto-4,3'-biphenyldiamine, N,N'-diisobutylidene-3,3'-dimercapto-4,4'-biphenyldiamine, N,N'-diisobutylidene-3,4'-dimercapto-4,3'-biphenyldiamine, N,N'-dicyclohexylidene-3,3'-dimercapto-4,4'-biphenyldiamine, N,N'-dicyclohexylidene-3,4'-dimercapto-4,3'-biphenyldiamine, N,N'-dicyclohexanomethylidene-3,3'-dimercapto-4,4'-biphenyldiamine, N,N'-dicyclohexanomethylidene-3,4'-dimercapto-4,3'-biphenyldiamine, N,N'-dibenzylidene-3,3'-dimercapto-4,4'-biphenyldiamine, and N,N'-dibenzylidene-3,4'-dimercapto-4,3'-biphenyldiamine.

In addition to the above compounds, the aromatic polyamine derivatives according to the present invention include compounds in which at least two of R^{a}, R^{b}, R^{c} and R^{d} are combined to form a ring. Examples of such aromatic polyamine derivatives include compounds whose amino groups are protected by the protecting group capable of protecting plural amino groups simultaneously (polyfunctional protecting group). Representative examples of these compounds include compounds corresponding to 1,2,4,5-tetraaminobenzene, except with two amino groups in the molecule being protected by one oxalyl group [compounds of Formula (1) in which Ring Z is a benzene ring or biphenyl ring; R^{a} and R^{c} are amino groups protected by one oxalyl group; and R^{b} and R^{d} are amino groups protected by one oxalyl group], and compounds corresponding to 1,2,4,5-tetraaminobenzene, except being protected by two butane-2,3-diylidene groups [compounds of Formula (1) in which Ring Z is a benzene ring or biphenyl ring; R^{a} and R^{c} are amino groups protected by one butane-2,3-diylidene group; and R^{b} and R^{d} are amino groups protected by one butane-2,3-diylidene group].

The aromatic polyamine derivatives represented by Formula (1) can be prepared, for example, by dehydrating and condensing an aromatic amine compound of Formula (1), in which at least one of R^{a}, R^{b}, R^{c}, and R^{d} is an amino group, with a ketone or aldehyde corresponding to a protecting group to protect the amino group at room temperature or with heating.

Acid catalysts can be used in the reaction. Examples of the acid catalysts include inorganic acids such as sulfuric acid and hydrochloric acid; organic acids such as acetic acid, p-toluenesulfonic acid, and methanesulfonic acid; Lewis acids such as boron fluoride-ether complex; and resins such as acidic ion-exchange resins. The amount of the acid catalyst can be set according to the type of acid and is, for example, about 0 to about 70 percent by mole, and preferably about 0 to about 60 percent by mole to the aromatic polyamine.

Dehydration can be carried out, for example, by a method in which an azeotropic solvent such as toluene is used, the solvent is separated from azeotropic water using a Dean-Stark water separator, and the solvent alone is refluxed; a method in which a solvent is refluxed in a Soxhlet extractor containing a desiccating agent such as anhydrous magnesium sulfate or molecular sieves; or a method in which dehydration is carried in the coexistence of a dehydrating agent, such as dicyclohexylcarbodiimide (DCC), in the reaction system.

The reaction can be carried out in the presence of, or in the absence of a solvent. The solvent is not specifically limited, as long as it does not adversely affect the reaction. Examples thereof include aromatic hydrocarbons such as benzene, toluene, and xylenes; aliphatic hydrocarbons such as hexane; alicyclic hydrocarbons such as cyclohexane; halogenated hydrocarbons such as methylene chloride; and aprotic polar solvents such as dimethylformamide and dimethylacetamide. The amount of the ketone or aldehyde is generally 1 mole or more per 1 mole of the amino group to be protected of the material aromatic amine compound. The ketone or aldehyde can be used in large excess. The reaction temperature can be set according typically to the types of reaction materials within a range of, for example, about 10°C to about 150°C, and preferably about 15°C to about 120°C. The reaction can be carried out at atmospheric pressure (normal pressure), under a pressure (under a load), or under reduced pressure. The reaction may be conducted in a nitrogen atmosphere and can be carried out according to a conventional system such as a batch system, a semi-batch system, or a continuous system.

The above-mentioned reaction yields an aromatic polyamine derivative represented by Formula (1) corresponding to the material aromatic amine compound. The reaction of 1,2,4,5-benzenetetramine and acetone yields, for example, N,N',N",N"'-tetraisopropylidene-1,2,4,5-benzenetetramine, and the reaction of 3,4,3',4'-biphenyltetramine and methyl ethyl ketone yields, for example, N,N',N",N'"-tetraisobutylidene-3,4,3',4'-biphenyltetramine. When the material aromatic amine compound has plural amino groups, the number of amino group(s) protected by an alkylidene group can be controlled by adjusting, for example, the amount of the ketone or aldehyde, and the reaction temperature and the reaction time.

After the completion of reaction, reaction products can be separated and purified by a separation procedure such as filtration, concentration, distillation, extraction, crystallization, recrystallization, adsorption, or chromatography, or a combination of these procedures. A target compound can be purified, for example, by removing unreacted ketone or aldehyde under reduced pressure, removing a residual acid component using typically an alkali when an acid catalyst is used, and carrying out, for example, distillation, recrystallization, or column chromatography.

The aromatic polyamine derivatives according to the present invention are useful as materials for forming dielectric films that comprise polybenzazoles having high heat resistance and low dielectric constants and are useful as semiconductor components.

The dielectric films comprising polybenzazoles can be formed by dissolving the aromatic polyamine derivative and the adamantanepolycarboxylic acid or a derivative thereof in an organic solvent to yield a material for forming dielectric films comprising a polymerizable composition, applying the material to a base material, and heating and thereby carrying out a polymerization reaction. In this procedure, each of the aromatic polyamine derivatives can be used alone or in combination.

The adamantanepolycarboxylic acid or a derivative thereof can be a compound represented by following Formula (2) or (2a): 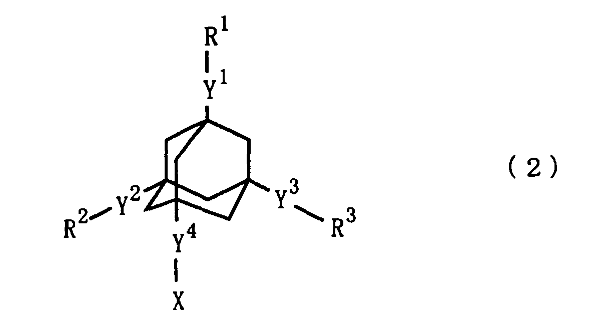 wherein X represents a hydrogen atom, a hydrocarbon group, or R⁴; R¹, R², R³ and R⁴ are the same as or different from each other and each represent a protected or unprotected carboxyl group, or a carbonyl halide group; and Y¹, Y², Y³, and Y⁴ are the same as or different from each other and each represent a single bond or a divalent aromatic cyclic group, wherein at least one of R¹, R², and R³ represents a protected carboxyl group or a carbonyl halide group (haloformyl group) when X is a hydrogen atom or a hydrocarbon group, and wherein at least one of R¹, R², R³, and R⁴ represents a protected carboxyl group or a carbonyl halide group when X is R⁴, 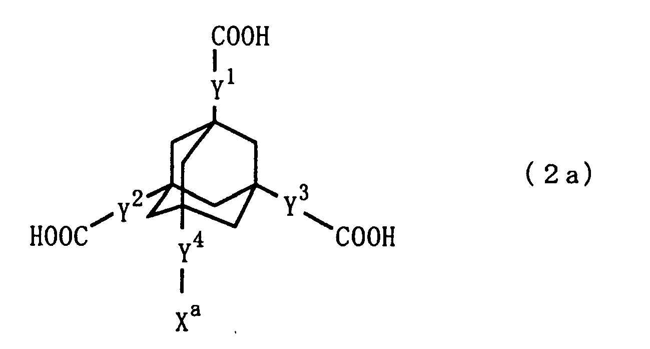 wherein X^{a} represents a hydrogen atom, a carboxyl group, or a hydrocarbon group; and Y¹, Y², Y³, and Y⁴ are the same as or different from each other and each represent a single bond or a divalent aromatic cyclic group.

In the formula, the hydrocarbon group in X includes aliphatic hydrocarbon groups, alicyclic hydrocarbon groups, aromatic hydrocarbon groups, and groups comprising these groups combined with each other. Examples of the aliphatic hydrocarbon groups include linear or branched-chain alkyl groups having about one to about twenty carbon atoms, such as methyl, ethyl, propyl, isopropyl, butyl, isobutyl, s-butyl, t-butyl, pentyl, hexyl, decyl, and dodecyl groups, of which those having about one to about ten carbon atoms are preferred, and those having about one to about six carbon atoms are more preferred; linear or branched-chain alkenyl groups having about two to about twenty carbon atoms, such as vinyl, allyl, 1-butenyl, and 3-methyl-4-pentenyl groups, of which those having about two to about ten carbon atoms are preferred, and those having about two to about five carbon atoms are more preferred; and linear or branched-chain alkynyl groups having about two to about twenty carbon atoms, such as ethynyl, propynyl, 1-butynyl, and 2-butynyl groups, of which those having about two to about ten carbon atoms are preferred, and those having about two to about five carbon atoms are more preferred.

Examples of the alicyclic hydrocarbon groups include monocyclic alicyclic hydrocarbon groups including cycloalkyl groups having about three to about twenty members, such as cyclopropyl, cyclobutyl, cyclopentyl, cyclohexyl, and cyclooctyl groups, of which those having about three to about fifteen members are preferred, and those having about three to about twelve members are more preferred, and cycloalkenyl groups having about three to about twenty members, such as cyclopropenyl, cyclobutenyl, cyclopentenyl, and cyclohexenyl groups, of which those having about three to about fifteen members are preferred, and those having about three to about ten members are more preferred; and bridged hydrocarbon groups comprising bridged carbon rings comprising about two to about four rings, such as adamantane ring, perhydroindene ring, decalin ring, perhydrofluorene ring, perhydroanthracene ring, perhydrophenanthrene ring, tricyclo[5.2.1.0^{2,6}]decane ring, perhydroacenaphthene ring, perhydrophenalene ring, norbornane ring, and norbornene ring. Examples of the aromatic hydrocarbon groups include aromatic hydrocarbon groups having about six to about twenty carbon atoms, such as phenyl, and naphthyl groups, of which those having about six to about fourteen carbon atoms are preferred.

The hydrocarbon groups comprising an aliphatic hydrocarbon group and an alicyclic hydrocarbon group combined with each other include cycloalkyl-alkyl groups including (C₃₋₂₀ cycloalkyl)-(C₁₋₄ alkyl) groups such as cyclopentylmethyl, cyclohexylmethyl, and 2-cyclohexylethyl groups. The hydrocarbon groups comprising an aliphatic hydrocarbon group and an aromatic hydrocarbon group combined with each other include aralkyl groups such as aralkyl groups having seven to eighteen carbon atoms; and alkylsubstituted aryl groups such as phenyl group or naphthyl group substituted with about one to about four (C₁₋₄ alkyl) groups.

The aliphatic hydrocarbon groups, alicyclic hydrocarbon groups, aromatic hydrocarbon groups, and groups comprising these groups combined with each other may each have one or more substituents. The substituents are not specifically limited, as long as they do not adversely affect the reaction. Examples of such substituents are halogen atoms such as fluorine, chlorine, bromine, and iodine; substituted oxy groups including alkoxy groups such as methoxy, and ethoxy groups, cycloalkyloxy groups, aryloxy groups, acyloxy groups, and silyloxy groups; substituted oxycarbonyl groups such as alkyloxycarbonyl groups and aryloxycarbonyl groups; acyl groups including acetyl group and other aliphatic acyl groups, acetoacetyl group, alicyclic acyl groups, and aromatic acyl groups; aliphatic hydrocarbon groups; alicyclic hydrocarbon groups; aromatic hydrocarbon groups; and heterocyclic groups.

When X is a hydrogen atom or a hydrocarbon group, the adamantanepolycarboxylic acids and derivatives thereof represented by Formula (2) or (2a) represent trifunctional adamantane compounds having functional groups (carboxyl group or equivalent groups thereto) at the 1-, 3-, and 5-positions of the adamantane skeleton. They represent tetrafunctional adamantane compounds having functional groups (carboxyl group or equivalent groups thereto) at the 1-, 3-, 5-, and 7-positions of the adamantane skeleton when X is R⁴, namely, when X is a protected or unprotected carboxyl group or a carbonyl halide group.

In the present invention, X is preferably a hydrogen atom, an alkyl group having one to six carbon atoms, an aromatic hydrocarbon group having six to fourteen carbon atoms, or R⁴. The substituent X is especially preferably R⁴, since the resulting compound is a tetrafunctional adamantane compound which is more highly crosslinkable.

Examples of the carboxyl-protecting groups in R¹ to R⁴ of Formula (2) include alkoxy groups including alkoxy groups having one to ten carbon atoms, such as methoxy, ethoxy, propoxy, isopropoxy, butoxy, isobutoxy, s-butoxy, t-butoxy, and hexyloxy, and (C₁₋₄ alkoxy)₁₋₂-(C₁₋₄ alkoxy) groups such as methoxymethyloxy, and methoxyethoxymethyloxy groups; cycloalkyloxy groups including cycloalkyloxy groups having three to twenty carbon atoms, such as cyclopentyloxy and cyclohexyloxy; tetrahydrofuranyloxy group; tetrahydropyranyloxy group; aryloxy groups including aryloxy groups having six to twenty carbon atoms, such as phenoxy, and methylphenoxy groups; aralkyloxy groups including aralkyloxy groups having seven to eighteen carbon atoms, such as benzyloxy, and diphenylmethyloxy groups; trialkylsilyloxy groups including tri-(C₁₋₄ alkyl)-silyloxy groups such as trimethylsilyloxy, and triethylsilyloxy groups; substituted or unsubstituted amino groups including amino group, mono- or di-(C₁₋₆alkyl)-amino groups such as methylamino, dimethylamino, ethylamino, and diethylamino, and cyclic amino groups such as pyrrolidino, and piperidino groups; substituted or unsubstituted hydrazino groups including hydrazino group, N-phenylhydrazino group, alkoxycarbonylhydrazino groups (including (C₁₋₁₀ alkoxy)carbonylhydrazino groups such as t-butoxycarbonylhydrazino group), and aralkyloxycarbonylhydrazino groups (including (C₇₋₁₈ aralkyl)oxycarbonylhydrazino groups such as benzyloxycarbonylhydrazino group); and acyloxy groups including (C₁₋₁₀acyl)oxy groups such as acetoxy and propionyloxy groups. The carboxyl-protecting groups are not limited to these groups, and any other protecting groups used in the field of organic synthesis can be used.

The carbonyl halide groups include carbonyl chloride group, carbonyl bromide group, carbonyl fluoride group, and carbonyl iodide group.

The compounds represented by Formula (2) are adamantanepolycarboxylic acid esters when R¹, R², R³, and R⁴ are each, for example, an alkoxycarbonyl group or an aryloxycarbonyl group; they are adamantanepolycarboxylic acid amides when R¹, R², R³, and R⁴ are each a substituted or unsubstituted carbamoyl group; and they are adamantanepolycarbonyl halides when R¹, R², R³, and R⁴ are each a carbonyl halide group.

Preferred substituents R¹, R², R³, and R⁹ include carboxyl group, (C₁₋₆ alkoxy) -carbonyl groups, (C₁₋₄ alkoxy) ₁₋₂₋(C₁₋₄ alkoxy)-carbonyl groups, N-substituted carbamoyl groups, tetrahydropyranyloxycarbonyl group, tetrahydrofuranyloxycarbonyl group, aryloxycarbonyl groups, trialkylsilyloxycarbonyl groups, and carbonyl halide groups.

The aromatic rings corresponding to the divalent aromatic cyclic groups in Y¹, Y², Y³, and Y⁴ include monocyclic or polycyclic aromatic hydrocarbon rings and heteroaromatic rings. The monocyclic aromatic hydrocarbon rings include benzene ring. Examples of the polycyclic aromatic hydrocarbon rings include rings having a fused ring structure in which two or more aromatic rings each possess two or more atoms in common, such as naphthalene ring, anthracene ring, phenanthrene ring, and phenalene ring; and rings having a structure in which two or more aromatic rings are combined with the interposition of a linkage group such as single bond, or an alicyclic ring, such as biphenyl ring, biphenylene ring, and fluorene ring. The heteroaromatic rings include monocyclic or polycyclic heteroaromatic rings containing one or more hetero atoms such as oxygen atoms, sulfur atoms, and nitrogen atoms. Specific examples of the heteroaromatic rings are monocyclic rings such as furan ring, thiophene ring, pyridine ring, and picoline ring; and polycyclic rings such as quinoline ring, isoquinoline ring, acridine ring, and phenazine ring. These aromatic rings may each have one or more substituents. The substituents include those exemplified as the substituents which the hydrocarbon group in X may have.

Of the adamantanepolycarboxylic acids and derivatives thereof for use in the present invention, trifunctional adamantane compounds (adamantanetricarboxylic acids and derivatives thereof) include compounds in which all the three functional groups are carboxyl groups, compounds in which one functional group is a protected carboxyl group or a carbonyl halide group, compounds in which two functional groups are each a protected carboxyl group or a carbonyl halide group, and compounds in which all the three functional groups are each a protected carboxyl group or a carbonyl halide groups.

Representative examples of the trifunctional adamantane compounds in which all the three functional groups are carboxyl groups include 1,3,5-adamantanetricarboxylic acid, 7-methyl-1,3,5-adamantanetricarboxylic acid, 7-phenyl-1,3,5-adamantanetricarboxylic acid, 1,3,5-tris(4-carboxyphenyl)adamantane, 1,3,5-tris(4-carboxyphenyl)-7-methyladamantane, and 1,3,5-tris(4-carboxyphenyl)-7-phenyladamantane.

Representative examples of the trifunctional adamantane compounds in which one functional group is a protected carboxyl group or a carbonyl halide group include 1-methoxycarbonyl-3,5-adamantanedicarboxylic acid, 1-(t-butoxycarbonyl)-3,5-adamantanedicarboxylic acid, 1-tetrahydropyranyl (THP)-oxycarbonyl-3,5-adamantanedicarboxylic acid, 1-phenoxycarbonyl-3,5-adamantanedicarboxylic acid, 1-methoxymethyl (MEM)-oxycarbonyl-3,5-adamantanedicarboxylic acid, 1-trimethylsilyl (TMS)-oxycarbonyl-3,5-adamantanedicarboxylic acid, 1,3-dicarboxy-5-adamantanecarbonyl chloride, 1-diethylcarbamoyl-3,5-adamantanedicarboxylic acid, 1-pyrrolidinylcarbonyl-3,5-adamantanedicarboxylic acid, and 1,3-bis(4-carboxyphenyl)-5-(4-methoxycarbonylphenyl)adamantane.

Representative examples of the trifunctional adamantane compounds in which two functional groups are each a protected carboxyl group or a carbonyl halide group include 1,3-bis(methoxycarbonyl)-5-adamantanemonocarboxylic acid, 1,3-bis(t-butoxycarbonyl)-5-adamantanemonocarboxylic acid, 1,3-bis(tetrahydropyranyl (THP)-oxycarbonyl)-5-adamantanemonocarboxylic acid, 1,3-bis(phenoxycarbonyl)-5-adamantanemonocarboxylic acid, 1,3-bis(methoxymethyl (MEM)-oxycarbonyl)-5-adamantanemonocarboxylic acid, 1,3-bis(trimethylsilyl (TMS)-oxycarbonyl)-5-adamantanemonocarboxylic acid, 1-carboxy-3,5-adamantanedicarbonyl dichloride, 1,3-bis(diethylcarbamoyl)-5-adamantanemonocarboxylic acid, 1,3-bis(1-pyrrolidinylcarbonyl)-5-adamantanemonocarboxylic acid, and 1-(4-carboxyphenyl)-3,5-bis(4-methoxycarbonylphenyl)-adamantane.

Representative examples of the trifunctional adamantane compounds in which all the three functional groups are each a protected carboxyl group or a carbonyl halide group include 1,3,5-tris(methoxycarbonyl)adamantane, 1,3,5-tris(t-butoxycarbonyl)adamantane, 1,3,5-tris(tetrahydropyranyl (THP)-oxycarbonyl)adamantane, 1,3,5-tris(phenoxycarbonyl)adamantane, 1,3,5-tris(methoxymethyl (MEM)-oxycarbonyl)adamantane, 1,3,5-tris(trimethylsilyl (TMS)-oxycarbonyl)adamantane, 1,3,5-adamantanetricarbonyl trichloride, 1,3,5-tris(diethylcarbamoyl)adamantane, 1,3,5-tris(1-pyrrolidinylcarbonyl)adamantane, and 1,3,5-tris(4-methoxycarbonylphenyl)adamantane.

Of the adamantanepolycarboxylic acids and derivatives thereof for use in the present invention, tetrafunctional adamantane compounds (adamantanetetracarboxylic acid and derivatives thereof) include compounds in which all the four functional groups are carboxyl groups, compounds in which one functional group is a protected carboxyl group or a carbonyl halide group, compounds in which two functional groups are each a protected carboxyl group or a carbonyl halide group, compounds in which three functional groups are each a protected carboxyl group or a carbonyl halide group, and compounds in which all the four functional groups are each a protected carboxyl group or a carbonyl halide group.

Representative examples of the tetrafunctional adamantane compounds in which all the four functional groups are carboxyl groups include 1,3,5,7-adamantanetetracarboxylic acid and 1,3,5,7-tetrakis(4-carboxyphenyl)adamantane. Representative examples of the tetrafunctional adamantane compounds in which one functional group is a protected carboxyl group or a carbonyl halide group include 1-methoxycarbonyl-3,5,7-adamantanetricarboxylic acid, 1-(t-butoxycarbonyl)-3,5,7-adamantanetricarboxylic acid, 1-tetrahydropyranyl (THP)-oxycarbonyl-3,5,7-adamantanetricarboxylic acid, 1-phenoxycarbonyl-3,5,7-adamantanetricarboxylic acid, 1-methoxymethyl (MEM)-oxycarbonyl-3,5,7-adamantanetricarboxylic acid, 1-trimethylsilyl (TMS)-oxycarbonyl-3,5,7-adamantanetricarboxylic acid, 1,3,5-tricarboxy-7-adamantanecarboxylic acid chloride, 1-diethylcarbamoyl-3,5,7-adamantanetricarboxylic acid, 1-(1-pyrrolidinylcarbonyl)-3,5,7-adamantanetricarboxylic acid, and 1,3,5-tris(4-carboxyphenyl)-7-(4-methoxycarbonylphenyl)adamantane.

Representative examples of the tetrafunctional adamantane compounds in which two functional groups are each a protected carboxyl group or a carbonyl halide group include 1,3-bis(methoxycarbonyl)-5,7-adamantanedicarboxylic acid, 1,3-bis(t-butoxycarbonyl)-5,7-adamantanedicarboxylic acid, 1,3-bis(tetrahydropyranyl (THP)-oxycarbonyl)-5,7-adamantanedicarboxylic acid, 1,3-bis(phenoxycarbonyl)-5,7-adamantanedicarboxylic acid, 1,3-bis(methoxymethyl (MEM)-oxycarbonyl)-5,7-adamantanedicarboxylic acid, 1,3-bis(trimethylsilyl (TMS)-oxycarbonyl)-5,7-adamantanedicarboxylic acid, 1,3-dicarboxy-5,7-adamantanedicarbonyl dichloride, 1,3-bis(diethylcarbamoyl)-5,7-adamantanedicarboxylic acid, 1,3-bis(1-pyrrolidinylcarbonyl)-5,7-adamantanedicarboxylic acid, and 1,3-bis(4-carboxyphenyl)-5,7-bis(4-methoxycarbonylphenyl)adamantane.

Representative examples of the tetrafunctional adamantane compounds in which three functional groups are each a protected carboxyl group or a carbonyl halide group include 1,3,5-tris(methoxycarbonyl)-7-adamantanemonocarboxylic acid, 1,3,5-tris(t-butoxycarbonyl)-7-adamantanemonocarboxylic acid, 1,3,5-tris(tetrahydropyranyl (THP)-oxycarbonyl)-7-adamantanemonocarboxylic acid, 1,3,5-tris(phenoxycarbonyl)-7-adamantanemonocarboxylic acid, 1,3,5-tris(methoxymethyl (MEM)-oxycarbonyl)-7-adamantanemonocarboxylic acid, 1,3,5-tris(trimethylsilyl (TMS)-oxycarbonyl)-7-adamantanemonocarboxylic acid, 1-carboxy-3,5,7-adamantanetricarbonyl trichloride, 1,3,5-tris(diethylcarbamoyl)-7-adamantanemonocarboxylic acid, 1,3,5-tris(1-pyrrolidinylcarbonyl)-7-adamantanemonocarboxylic acid, and 1-(4-carboxyphenyl)-3,5,7-tris(4-methoxycarbonylphenyl)adamantane.

Representative examples of the tetrafunctional adamantane compounds in which all the four functional groups are each a protected carboxyl group or a carbonyl halide group include 1,3,5,7-tetrakis(methoxycarbonyl)adamantane, 1,3,5,7-tetrakis(t-butoxycarbonyl)adamantane, 1,3,5,7-tetrakis(tetrahydropyranyl (THP)-oxycarbonyl)adamantane, 1,3,5,7-tetrakis(phenoxycarbonyl)adamantane, 1,3,5,7-tetrakis(methoxymethyl (MEM)-oxycarbonyl)adamantane, 1,3,5,7-tetrakis(trimethylsilyl (TMS)-oxycarbonyl)adamantane, 1,3,5,7-adamantanetetracarbonyl tetrachloride, 1,3,5,7-tetrakis(diethylcarbamoyl)adamantane, 1,3,5,7-tetrakis(1-pyrrolidinylcarbonyl)adamantane, and 1,3,5,7-tetrakis(4-methoxycarbonylphenyl)adamantane.

Each of these adamantanepolycarboxylic acids and adamantanecarboxylic acid derivatives can be used alone or in combination.

The adamantanepolycarboxylic acids represented by Formula (2) or (2a) can be prepared by a known or conventional method or by using a known organic synthesis reaction.

The material for forming dielectric films may include one or more other components than the above-mentioned components. For example, the material may further comprise small amounts of any of catalysts for accelerating the polymerization reaction, including acid catalysts such as sulfuric acid; thickening agents for increasing the viscosity of a solution (composition), such as ethylene glycol; monocarboxylic acids for adjusting the molecular weight after polymerization, such as adamantanecarboxylic acid; dicarboxylic acids for adjusting the degree of crosslinking after polymerization, such as adamantanedicarboxylic acid; and adhesion promoters for increasing the adhesion of the resulting dielectric film with a substrate, such as trimethoxyvinylsilane.

The solvent is not specifically limited, as long as it does not adversely affect the reaction for forming a ring between an adamantanepolycarboxylic acid and an aromatic polyamine derivative. Examples of such solvents include aliphatic hydrocarbons such as hexane, heptane, and octane; alicyclic hydrocarbons such as cyclohexane and methylcyclohexane; aromatic hydrocarbons such as benzene, toluene, xylenes, ethylbenzene, and mesitylene; halogenated hydrocarbons such as dichloromethane, dichloroethane, chloroform, and carbon tetrachloride; alcohols such as methanol, ethanol, propanol, butanol, and ethylene glycol; ethers such as dioxane, tetrahydrofuran, diethyl ether, and propylene glycol monomethyl ether (PGME); esters such as formic esters, acetic esters, propionic esters, benzoic esters, γ-butyrolactone, and propylene glycol monomethyl ether acetate (PGMEA); ketones such as acetone, methyl ethyl ketone, diethyl ketone, methyl isobutyl ketone, cyclopentanone, and cyclohexanone; carboxylic acids such as formic acid, acetic acid, propionic acid, and butyric acid; aprotic polar solvents including nitriles such as acetonitrile, propionitrile, and benzonitrile, amides such as formamide, dimethylformamide, acetamide, dimethylacetamide, and N-methylpyrrolidone, and sulfoxides such as dimethyl sulfoxide. Each of these solvents can be used alone or in combination.

The polymerizable composition constituting the material for forming dielectric films can be prepared by any method, as long as the adamantanepolycarboxylic acid and the aromatic polyamine derivative (monomer component) can be fully dissolved in a solvent. For example, it can be prepared, for example, by stirring a mixture comprising the monomer components, the solvent, and other components or leaving the mixture stand. The ratio of the adamantanepolycarboxylic acid to the aromatic polyamine derivative in the mixture can be any ratio according to the solubility in the used solvent, as long as the functions of the resulting dielectric film are not affected. The ratio (molar ratio) of the adamantanepolycarboxylic acid to the aromatic polyamine derivative is preferably about 10/90 to about 60/40, and more preferably about 20/80 to about 50/50.

The total concentration of the adamantanepolycarboxylic acid and the aromatic polyamine derivative (concentration of total monomers) to the solvent is arbitrary selected according to the solubility in the used solvent and is, in terms of the total concentration of monomers, for example, about 5 to about 70 percent by weight, and preferably about 10 to about 60 percent by weight. The material for forming dielectric films containing the aromatic polyamine derivative can dissolve monomer components in higher concentrations due to an improved solubility of the aromatic polyamine derivative in a solvent. The dielectric films formed from the material for forming dielectric films containing monomer components in higher concentrations can have an increased film thickness and exhibit excellent electric properties. Thus, dielectric films having a variety of film thickness corresponding to various semiconductor production processes can be formed.

The dissolution is conducted, for example, in an atmosphere of the air, as long as the aromatic polyamine derivative is not oxidized. The dissolution is preferably conducted in an atmosphere of an inert gas such as nitrogen or argon. The dissolution temperature is not specifically limited, and heating may be conducted according to the solubilities of the monomers, and the boiling point of the solvent. For example, the dissolution temperature is about 0°C to about 200°C, and preferably about 10°C to about 150°C.

A possible candidate for the material for forming dielectric films is a polycondensation product (polybenzazole) between an adamantanepolycarboxylic acid and an aromatic polyamine derivative, so as to yield a dielectric film that can exhibit high heat resistance as a result of high degree of crosslinking. Such a polybenzazole, however, has a low solubility in a solvent due to its high degree of crosslinking and is difficult to be used as a material for forming dielectric films by coating. In contrast, dielectric films comprising highly crosslinked polybenzazoles can be easily formed from the material for forming dielectric films containing the aromatic polyamine derivative, by applying the material as intact to a base material and carrying out polymerization thereafter, since the monomer components in the material are completely dissolved in the solvent.

The base material to which the material for forming dielectric films is applied includes, for example, silicon wafers, metal substrates, and ceramic substrates. The coating procedure is not specifically limited and can be any conventional procedure such as spin coating, dip coating, or spraying.

The heating temperature is not specifically limited, as long as polymerizable components to be used can be polymerized, and is, for example, about 100°C to about 500°C, and preferably about 150°C to about 450°C. The heating can be conducted at a constant temperature or with a stepwise temperature gradient. The heating can be carried out, for example, in an atmosphere of the air, as long as the properties of the resulting thin film are not adversely affected, and is preferably carried out in an atmosphere of an inert gas such as nitrogen or argon gas or in a vacuum atmosphere.

As a result of the heating, the adamantanepolycarboxylic acid and the aromatic polyamine derivative in the material for forming dielectric films undergo polycondensation, generally accompanied with elimination of carboxyl-protecting group(s) and/or amino-protecting group(s), to thereby yield a polybenzazole containing an adamantane skeleton, such as an imidazole, oxazole, or thiazole, as a polymerization product.

The dielectric film formed as a result of heating comprises, as main constitutive units, an adamantane ring, an aromatic ring, and an azole ring or a six-membered nitrogen-containing ring (a ring formed in polycondensed portion) contained in the polymer formed from the material for forming dielectric films. By using, for example, an adamantanepolycarboxylic acid having three functional groups, the dielectric film can be a highly crosslinked polymer film, in which the adamantane compound having a three dimensional structure and an aromatic polyamide having a two-dimensional structure are combined to form a structure being crosslinked in three directions with the adamantane skeleton as apexes (crosslinking points). This structure comprises a unit comprising three hexagons each possessing two apexes or two sides in common. By using an adamantanepolycarboxylic acid having four functional groups, the dielectric film can be a network polymer film which has a structure being crosslinked in four directions with the adamantane skeleton as apexes (crosslinking points). This structure comprises a unit comprising three hexagons each possessing two sides in common. These dielectric films can have excellent relative dielectric constants, since they contain a large number of uniformly dispersed molecular-scale pores.

The film thickness of the dielectric film formed as a result of heating is, for example, about 50 nm or more (about 50 to about 2000 nm), preferably about 100 nm or more (about 100 to about 2000 nm), and more preferably about 300 nm or more (about 300 to about 2000 nm). The above material for forming dielectric films can yield a coating composition having a high monomer concentration, and the film thickness as specified above can be obtained even in dielectric films comprising polybenzazoles. If the thickness is less than 50 nm, electric properties may be adversely affected due typically to leakage current, or the film may not be significantly smoothened by chemical mechanical polishing (CMP) in semiconductor production processes. Due to these and other problems, the resulting film is not suitable as an interlayer dielectric film.

The above-mentioned dielectric films can form dielectric films having low dielectric constants and high heat resistance. The dielectric films can be used, for example, as dielectric films (insulating films) in electronic materials and parts typically for semiconductor devices.

### Examples

The present invention will be illustrated in further detail with reference to several Examples below which by no means limit the scope of the present invention. The symbols "s", "m", and "w" in infrared absorption spectral data indicate the absorption intensities of wavelengths described in front of the symbols and mean "strong", "moderate", and "weak" absorptions, respectively. The film thickness of a sample polymer film was determined using an ellipsometer.

### Synthesis Example 1

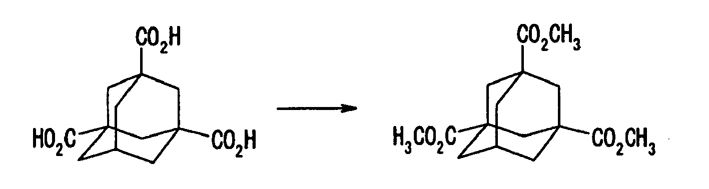

### Synthesis of 1,3,5-adamantanetricarboxylic acid trimethyl ester

In a 200-ml flask equipped with a stirrer, a condenser, and a thermometer were placed 26.8 g (100 mmol) of 1,3,5-adamantanetricarboxylic acid, 100 ml of methanol, and 0.49 g (5 mmol) of sulfuric acid, followed by heating under reflux in a nitrogen atmosphere for three hours. After cooling this to room temperature, methanol was removed therefrom under reduced pressure, the residual reaction mixture was dissolved in ethyl acetate, the solution was washed with a 10% aqueous sodium carbonate solution and water to remove a residual acid component. Ethyl acetate was removed from the ethyl acetate solution under reduced pressure, to thereby yield 27.3 g (88 mmol) of 1,3,5-adamantanetricarboxylic acid trimethyl ester in a yield of 88%.
[NMR spectral data]
¹H-NMR (CDCl₃) δ (ppm) : 1.84 (m, 6H), 2.01 (m, 6H), 2.30 (m, 1H), 3. 65 (m, 9H)
¹³C- NMR (CDCl₃) δ (ppm): 27.86, 37.06, 39.11, 41.28, 51.91, 176.40

### Example 1

### Synthesis of 3,3'-diaminobenzidinetetraisopropanoimine [N,N',N",N'"-tetraisopropylidene-3,4,3',4'-biphenyltetramine]

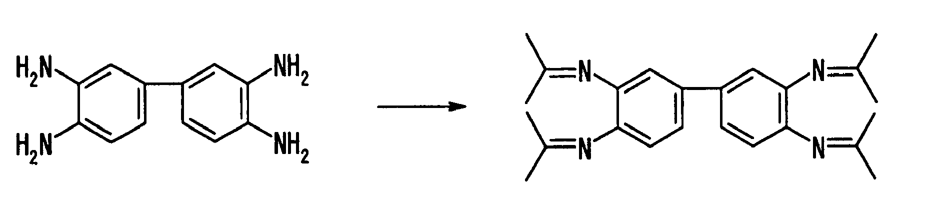

In a 200-ml flask equipped with a Soxhlet extractor containing Molecular Sieves 4A as a dehydrating agent, a stirrer, a condenser, and a thermometer were placed 21.4 g (100 mmol) of 3,3'-diaminobenzidine, 100 ml of acetone, and 0.95 g (5 mmol) of p-toluenesulfonic acid monohydrate, followed by heating under reflux in a nitrogen atmosphere for three hours. After cooling this to room temperature, acetone was removed therefrom under reduced pressure, the residue was diluted with ethyl acetate and washed with water and a 10% aqueous sodium carbonate solution to thereby remove a residual acid component. After removing ethyl acetate from this solution under reduced pressure, the residue was purified by silica gel chromatography and thereby yielded 28.5 g (76 mmol) of target 3,3'-diaminobenzidinetetraisopropanoimine [N,N',N",N'"-tetraisopropylidene-3,4,3',4'-biphenyltetramine] in a yield of 76%.
Infrared absorption spectral data (cm⁻¹): 1627 (C=N)
MS: 375 (M+H), 359, 333, 324

### Example 2

### Synthesis of 3,3'-diaminobenzidinemonoisopropanoimine [N'-isopropylidene-3,4,3',4'-biphenyltetramine] and 3,3'-diaminobenzidinediisopropanoimine [N',N"'-diisopropylidene-3,4,3',4'-biphenyltetramine]

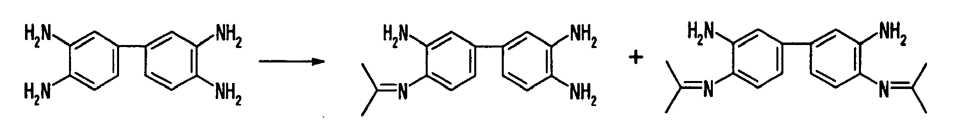

In a 200-ml flask equipped with a Soxhlet extractor containing Molecular Sieves 4A as a dehydrating agent, a stirrer, a condenser, and a thermometer were placed 21.4 g (100 mmol) of 3,3'-diaminobenzidine, 100 ml of acetone, and 3.0 g (50 mmol) of acetic acid, followed by heating under reflux in a nitrogen atmosphere for three hours. After cooling this to room temperature, acetone was removed therefrom under reduced pressure, the residue was purified by silica gel chromatography and thereby yielded 16.5 g (65 mmol) of target 3,3'-diaminobenzidinemonoisopropanoimine [N'-isopropylidene-3,4,3',4'-biphenyltetramine] and 4.7 g (16 mmol) of 3,3'-diaminobenzidinediisopropanoimine [N',N"'-diisopropylidene-3,4,3',4'-biphenyltetramine].
[Spectral data of 3,3'-diaminobenzidinemonoisopropanoimine]
Infrared absorption spectral data (cm⁻¹): 1628 (C=N)
MS: 255 (M+H), 238, 198
[Spectral data of 3,3'-diaminobenzidinediisopropanoimine]
Infrared absorption spectral data (cm⁻¹): 1627 (C=N)
MS: 295 (M+H), 278, 238

### Example 3

### Synthesis of 3,3'-diaminobenzidinediisobutylimine [N, N' , N" , N"'-tetraisobutylidene-3, 4, 3' , 4'-biphenyltetramine]

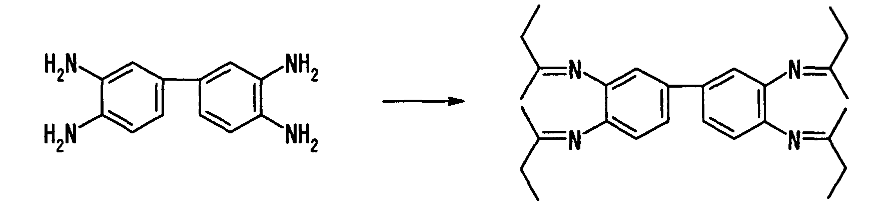

In a 200-ml flask equipped with a Soxhlet extractor containing Molecular Sieves 4A as a dehydrating agent, a stirrer, a condenser, and a thermometer were placed 21.4 g (100 mmol) of 3,3'-diaminobenzidine, 100 ml of methyl ethyl ketone, and 0.95 g (5 mmol) of p-toluenesulfonic acid monohydrate, followed by stirring at 60°C in a nitrogen atmosphere for five hours. After cooling this to room temperature, methyl ethyl ketone was removed therefrom under reduced pressure, the residue was diluted with ethyl acetate, and was washed with water and a 10% aqueous sodium carbonate solution to thereby remove a residual acid component. After removing ethyl acetate from this solution under reduced pressure, the residue was purified by silica gel chromatography and thereby yielded 30.1 g (70 mmol) of target 3,3'-diaminobenzidinetetraisobutylimine [N,N', N", N"'-tetraisobutylidene-3,4,3',4'-biphenyltetramine].
Infrared absorption spectral data (cm⁻¹): 1630 (C=N)
MS: 431 (M+H), 375

### Example 4

### Synthesis of 3,3'-diaminobenzidinetetracyclohexanomethylimine [N,N',N",N"'-tetracyclohexanomethylidene-3,4,3',4'-biphenyltetramine]

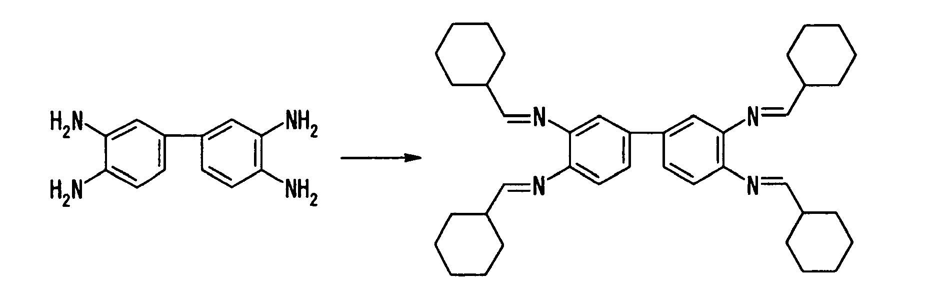

In a 200-ml flask equipped with a Dean-Stark apparatus, a stirrer, a condenser, and a thermometer were placed 21.4 g (100 mmol) of 3,3'-diaminobenzidine, 100 ml of toluene, 67.3 g (600 mmol) of cyclohexanecarbaldehyde, and 0.95 g (5 mmol) of p-toluenesulfonic acid monohydrate, followed by heating under reflux in a nitrogen atmosphere for eight hours. After cooling this to room temperature, toluene was removed therefrom under reduced pressure, the residue was diluted with ethyl acetate and was washed with water and a 10% aqueous sodium carbonate solution to thereby remove a residual acid component. After removing ethyl acetate from this solution under reduced pressure, the residue was purified by silica gel chromatography and thereby yielded 42.5 g (72 mmol) of target 3,3'-diaminobenzidinetetracyclohexanomethylimine [N,N',N",N'"-tetracyclohexanomethylidene-3,4,3',4'-biphenyltetramine].
Infrared absorption spectral data (cm⁻¹): 1629 (C=N)
MS: 591 (M+H), 495

### Example 5

### Synthesis of 3,3'-diaminobenzidinetetracyclohexanoimine [N,N',N",N'"-tetracyclohexylidene-3,4,3',4'-biphenyltetramine]

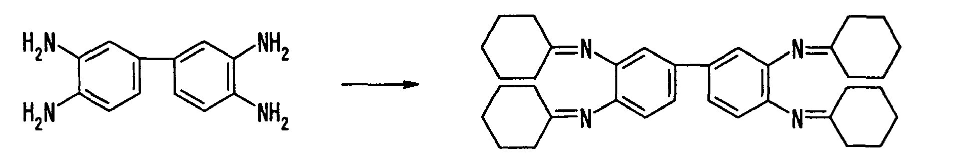

In a 200-ml flask equipped with a stirrer, a condenser, and a thermometer were placed 21.4 g (100 mmol) of 3,3'-diaminobenzidine and 100 ml of cyclohexanone, followed by refluxing at 60°C in a nitrogen atmosphere for two hours. After cooling this to room temperature, cyclohexanone was removed therefrom under reduced pressure, the residue was purified by silica gel chromatography and thereby yielded 48.1 g (90 mmol) of target 3,3'-diaminobenzidinetetracyclohexanoimine [N,N',N",N'"-tetracyclohexylidene-3,4,3',4'-biphenyltetramine].
Infrared absorption spectral data (cm⁻¹): 1636 (C=N)
MS: 535 (M+H), 491, 453

### Example 6

### Synthesis of 3,3'-dihydroxybenzidinediisopropanoimine [N,N'-diisopropylidene-3,3'-dihydroxybenzidine]

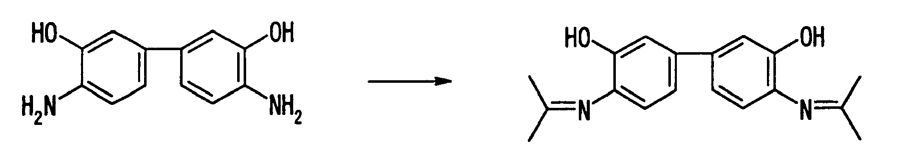

In a 300-ml flask equipped with a Soxhlet extractor containing Molecular Sieves 4A as a dehydrating agent, a stirrer, a condenser, and a thermometer were placed 21.6 g (100 mmol) of 3,3'-dihydroxybenzidine, 100 ml of acetone, 100 ml of dimethylacetamide, and 0.95 g (5 mmol) of p-toluenesulfonic acid monohydrate, followed by stirring at 60°C in a nitrogen atmosphere for eight hours. After cooling this to room temperature, acetone and dimethylacetamide were removed therefrom under reduced pressure, the residue was purified by silica gel chromatography and thereby yielded 21.3 g (72 mmol) of target 3,3'-dihydroxybenzidinediisopropanoimine [N,N'-diisopropylidene-3,3'-dihydroxybenzidine].
Infrared absorption spectral data (cm⁻¹): 1628 (C=N)
MS: 297 (M+H), 279, 240

### Example 7

### Synthesis of 3,3'-dihydroxybenzidinedicyclohexanoimine [N,N'-dicyclohexylidene-3,3'-dihydroxybenzidine]

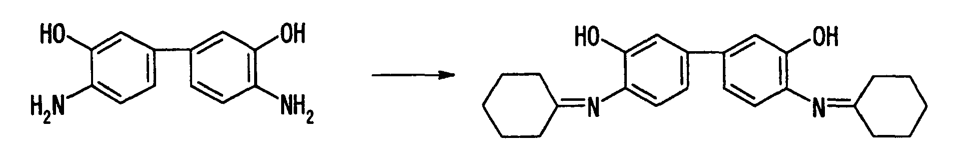

In a 300-ml flask equipped with a Soxhlet extractor containing Molecular Sieves 4A as a dehydrating agent, a stirrer, a condenser, and a thermometer were placed 21.6 g (100 mmol) of 3,3'-dihydroxybenzidine, 100 ml of cyclohexanone, 100 ml of dimethylacetamide, and 0.95 g (5 mmol) of p-toluenesulfonic acid monohydrate, followed by stirring at 60°C in a nitrogen atmosphere for eight hours. After cooling this to room temperature, cyclohexanone and dimethylacetamide were removed therefrom under reduced pressure, the residue was purified by silica gel chromatography and thereby yielded 32.0 g (85 mmol) of target 3,3'-dihydroxybenzidinedicyclohexanoimine [N,N'-dicyclohexylidene-3,3'-dihydroxybenzidine].
Infrared absorption spectral data (cm⁻¹): 1637 (C=N)
MS: 377 (M+H), 295

### Example 8

### Synthesis of 1,2,4,5-benzenetetraminetetraisopropanoimine [N,N',N",N'"-tetraisopropylidene-1,2,4,5-benzenetetramine]

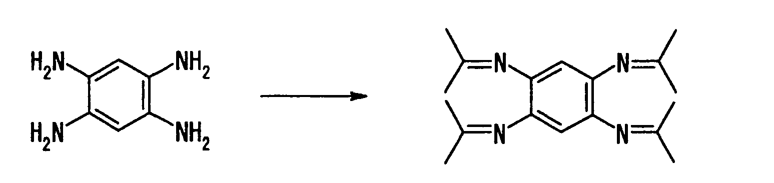

In a 200-ml flask equipped with a Soxhlet extractor containing Molecular Sieves 4A as a dehydrating agent, a stirrer, a condenser, and a thermometer were placed 13.8 g (100 mmol) of 1,2,4,5-benzenetetramine, 100 ml of acetone, and 0.95 g (5 mmol) of p-toluenesulfonic acid monohydrate, followed by heating under reflux in a nitrogen atmosphere for five hours. After cooling this to room temperature, acetone was removed therefrom under reduced pressure, the residue was diluted with ethyl acetate, and was washed with water and a 10% aqueous sodium carbonate solution to thereby remove a residual acid component. After removing ethyl acetate from this solution under reduced pressure, the residue was purified by silica gel chromatography and thereby yielded 20.9 g (70 mmol) of target 1,2,4,5-benzenetetraminetetraisopropanoimine [N,N',N",N'"-tetraisopropylidene-1,2,4,5-benzenetetramine].
Infrared absorption spectral data (cm⁻¹): 1628 (C=N)
MS: 299 (M+H), 257

### Example 9

### Synthesis of 1,2,4,5-benzenetetraminemonoisopropanoimine [N-isopropylidene-1,2,4,5-benzenetetramine] and 1,2,4,5-benzenetetraminediisopropanoimine [N,N"-diisopropylidene-1,2,4,5-benzenetetramine]

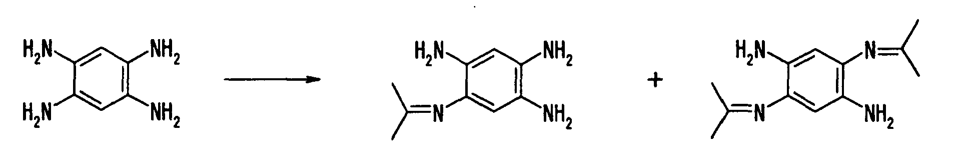

In a 200-ml flask equipped with a Soxhlet extractor containing Molecular Sieves 4A as a dehydrating agent, a stirrer, a condenser, and a thermometer were placed 13.8 g (100 mmol) of 1,2,4,5-benzenetetramine, 100 ml of acetone, and 3.0 g (50 mmol) of acetic acid, followed by heating under reflux in a nitrogen atmosphere for three hours. After cooling this to room temperature, acetone was removed therefrom under reduced pressure, the residue was purified by silica gel chromatography and thereby yielded 7.5 g (42 mmol) of target 1,2,4,5-benzenetetraminemonoisopropanoimine [N-isopropylidene-1,2,4,5-benzenetetramine] and 3.9 g (18 mmol) of 1,2,4,5-benzenetetraminediisopropanoimine [N,N"-diisopropylidene-1,2,4,5-benzenetetramine].
[Spectral data of 1,2,4,5-benzenetetraminemonoisopropanoimine]
Infrared absorption spectral data (cm⁻¹): 1628 (C=N)
MS: 179 (M+H), 137
[Spectral data of 1,2,4,5-benzenetetraminediisopropanoimine]
Infrared absorption spectral data (cm⁻¹): 1627 (C=N)
MS: 219 (M+H), 177

### Example 10

### Synthesis of 1,2,4,5-benzenetetraminetetracyclohexanoimine [N,N',N",N"'-tetracyclopropylidene-1,2,4,5-benzenetetramine]

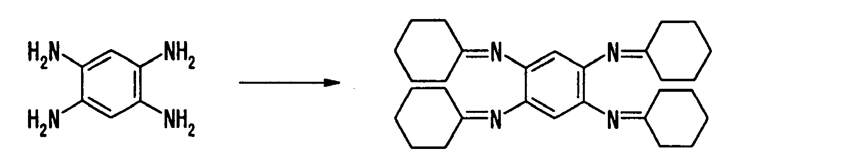

In a 200-ml flask equipped with a stirrer, a condenser, and a thermometer were placed 13.8 g (100 mmol) of 1,2,4,5-benzenetetramine and 100 ml of cyclohexanone, followed by stirring at 60°C in a nitrogen atmosphere for four hours. After cooling this to room temperature, cyclohexanone was removed therefrom under reduced pressure, the residue was purified by silica gel chromatography and thereby yielded 39.4 g (86 mmol) of target 1,2,4,5-benzenetetraminetetracyclohexanoimine [N,N',N",N"'-tetracyclopropylidene-1,2,4,5-benzenetetramine].
Infrared absorption spectral data (cm⁻¹): 1636 (C=N)
MS: 459 (M+H), 377

### Example 11

### Synthesis of 4,6-diaminoresorcinoldiisopropanoimine [N,N'-diisopropylidene-2,4-dihydroxy-1,5-benzenediamine]

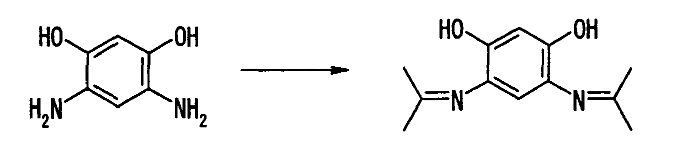

In a 300-ml flask equipped with a Soxhlet extractor containing Molecular Sieves 4A as a dehydrating agent, a stirrer, a condenser, and a thermometer were placed 14.0 g (100 mmol) of 4,6-diaminoresorcinol, 100 ml of acetone, 100 ml of dimethylacetamide, and 0.95 g (5 mmol) of p-toluenesulfonic acid monohydrate, followed by stirring at 60°C in a nitrogen atmosphere for six hours. After cooling this to room temperature, acetone and dimethylacetamide were removed therefrom under reduced pressure, the residue was purified by silica gel chromatography and thereby yielded 16.5 g (75 mmol) of target 4,6-diaminoresorcinoldiisopropanoimine [N,N'-diisopropylidene-2,4-dihydroxy-1,5-benzenediamine].
Infrared absorption spectral data (cm⁻¹): 1628 (C=N)
MS: 221 (M+H), 179

### Example 12

### Synthesis of 4,6-diaminoresorcinoldicyclohexanoimine [N,N'-dicyclohexylidene-2,4-dihydroxy-1,5-benzenediamine]

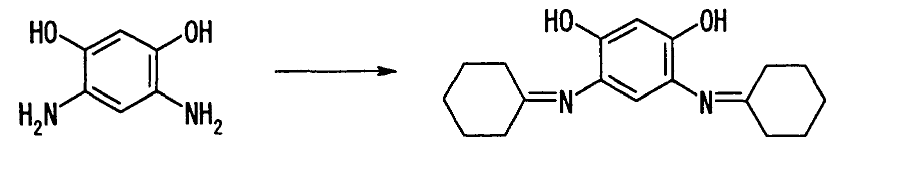

In a 300-ml flask equipped with a Soxhlet extractor containing Molecular Sieves 4A as a dehydrating agent, a stirrer, a condenser, and a thermometer were placed 14.0 g (100 mmol) of 4,6-diaminoresorcinol, 100 ml of cyclohexanone, 100 ml of dimethylacetamide, and 0.95 g (5 mmol) of p-toluenesulfonic acid monohydrate, followed by stirring at 60°C in a nitrogen atmosphere for six hours. After cooling this to room temperature, cyclohexanone and dimethylacetamide were removed therefrom under reduced pressure, the residue was purified by silica gel chromatography and thereby yielded 23.4 g (78 mmol) of target 4,6-diaminoresorcinoldicyclohexanoimine [N,N'-dicyclohexylidene-2,4-dihydroxy-1,5-benzenediamine].
Infrared absorption spectral data (cm⁻¹): 1638 (C=N)
MS: 301 (M+H), 219

### Example 13

### Synthesis of 1,3-dianilino-4,6-diaminobenzenediisopropanoimine [N,N"'-diisopropylidene-N',N" -diphenyl-1,2,4,5-benzenetetramine]

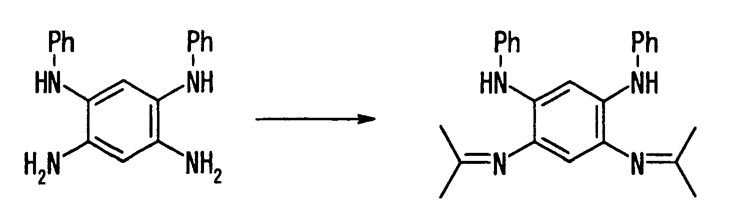

In a 300-ml flask equipped with a Soxhlet extractor containing Molecular Sieves 4A as a dehydrating agent, a stirrer, a condenser, and a thermometer were placed 29.0 g (100 mmol) of 1,3-dianilino-4,6-diaminobenzene, 100 ml of acetone, 100 ml of dimethylacetamide, and 0.95 g (5 mmol) of p-toluenesulfonic acid monohydrate, followed by stirring at 60°C in a nitrogen atmosphere for eight hours. After cooling this to room temperature, acetone and dimethylacetamide were removed therefrom under reduced pressure, the residue was purified by silica gel chromatography and thereby yielded 27.0 g (73 mmol) of target 1,3-dianilino-4,6-diaminobenzenediisopropanoimine [N,N'"-diisopropylidene-N',N"-diphenyl-1,2,4,5-benzenetetramine].
Infrared absorption spectral data (cm⁻¹): 1630 (C=N)
MS: 371 (M+H), 329

### Example 14

### Synthesis of 1,3-dianilino-4,6-diaminobenzenedicyclohexanoimine [N,N"'-dicyclohexylidene-N',N"-diphenyl-1,2,4,5-benzenetetramine]

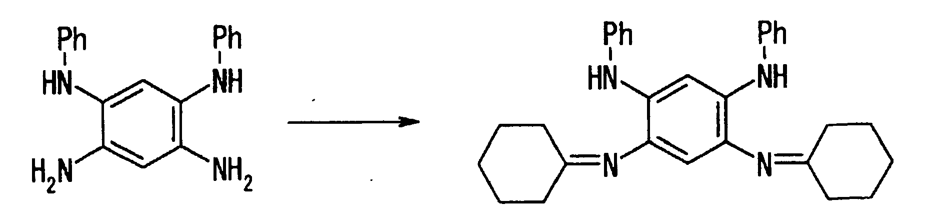

In a 300-ml flask equipped with a Soxhlet extractor containing Molecular Sieves 4A as a dehydrating agent, a stirrer, a condenser, and a thermometer were placed 29.0 g (100 mmol) of 1,3-dianilino-4,6-diaminobenzene, 100 ml of cyclohexanone, 100 ml of dimethylacetamide, and 0.95 g (5 mmol) of p-toluenesulfonic acid monohydrate, followed by stirring at 60°C in a nitrogen atmosphere for eight hours. After cooling this to room temperature, cyclohexanone.and dimethylacetamide were removed therefrom under reduced pressure, the residue was purified by silica gel chromatography and thereby yielded 31.5 g (70 mmol) of target 1,3-dianilino-4,6-diaminobenzenedicyclohexanoimine [N, N"' -dicyclohexylidene-N' , N"-diphenyl-1, 2, 4, 5-benzenetetramine].
Infrared absorption spectral data (cm⁻¹): 1639 (C=N)
MS: 451 (M+H), 369

### Example 15

In 100 g of mesitylene were dissolved 2.48 g (8 mmol) of 1,3,5-adamantanetricarboxylic acid trimethyl ester prepared in Synthesis Example 1 and 6.42 g (12 mmol) of 3,3'-diaminobenzidinetetracyclohexanoimine [N,N',N",N'"-tetracyclohexylidene-3,4,3',4'-biphenyltetramine] prepared in Example 5 at room temperature in a nitrogen atmosphere and thereby yielded a coating composition having a monomer concentration of 8.2 percent by weight. The coating composition was passed through a filter with a pore size of 0.1 micron and was applied to an 8-inch silicon wafer by spin coating. This was heated at 300°C in a nitrogen atmosphere for thirty minutes, was further heated at 400°C for thirty minutes, and thereby yielded a film. The infrared absorption spectrum of the resulting polymer film was determined to find that the target crosslinked polybenzimidazole film was formed as shown in the infrared absorption spectral data in FIG. 1. The film has a film thickness of 300 nm.
Infrared absorption spectral data (cm⁻¹):
805 (m), 1280 (m), 1403 (m), 1450 (s), 1522 (w), 1625 (w), 2857 (s), 2928 (s), 3419 (w)

### Comparative Example 1

The procedure of Example 15 was repeated, except for using the same amounts as in Example 15 of 1,3,5-adamantanetricarboxylic acid and 3,3'-diaminobenzidine instead of 1,3,5-adamantanetricarboxylic acid trimethyl ester and N,N',N",N'"-tetracyclohexylidene-3,4,3',4'-biphenyltetramine, respectively, to find the solubility in the solvent (mesitylene) was low. Consequently, a coating composition having a monomer concentration of 1.2 percent by weight was prepared by the procedure of Example 15, except for using 0.54 g (2 mmol) of 1,3,5-adamantanetricarboxylic acid and 0.64 g (3 mmol) of 3,3'-diaminobenzidine. By using this coating composition, a polymer film was prepared by the procedure of Example 5, and the infrared absorption spectrum of the polymer film was determined to find that the target crosslinked polybenzimidazole film was formed. The film has a film thickness of less than 20 nm.

### Example 16

A coating composition having a monomer concentration of 14.7 percent by weight was prepared by dissolving 4.42 g (12 mmol) of 1,3,5,7-adamantanetetracarboxylic acid tetramethyl ester and 12.83 g (24 mmol) of 3,3'-diaminobenzidinetetracyclohexanoimine [N,N',N",N'"-tetracyclohexylidene-3,4,3',4'-biphenyltetramine] prepared according to Example 5 in 100 g of methyl isobutyl ketone (MIBK) at room temperature in a nitrogen atmosphere. The coating composition was passed through a filter with a pore size of 0.1 micron and was applied to an 8-inch silicon wafer by spin coating. This was heated at 300°C in a nitrogen atmosphere for thirty minutes and further heated at 400°C for thirty minutes to yield a film. The infrared absorption spectrum of the resulting polymer film was determined to find that the target crosslinked polybenzimidazole film was formed. The film has a film thickness of 400 nm.

### Comparative Example 2

The procedure of Example 16 was repeated, except for using the same amounts as in Example 16 of 1,3,5,7-adamantanetetracarboxylic acid and 3,3'-diaminobenzidine instead of 1,3,5,7-adamantanetetracarboxylic acid tetramethyl ester and N,N',N " ,N " '-tetracyclohexylidene-3,4,3',4'-biphenyltetramine, respectively, to find that the solubility in the solvent (methyl isobutyl ketone: MIBK) was low. Consequently, a coating composition having a monomer concentration of 1.5 percent by weight was prepared by the procedure of Example 16, except for using 0.62 g (2 mmol) of 1,3,5,7-adamantanetetracarboxylic acid and 0.86 g (4 mmol) of 3,3'-diaminobenzidine. By using this coating composition, a polymer film was prepared by the procedure of Example 16, and the infrared absorption spectrum of the film was determined to find that the target crosslinked polybenzimidazole film was formed. The film has a film thickness of less than 20 nm.

### Example 17

A coating composition having a monomer concentration of 11.7 percent by weight was prepared by dissolving 4.29 g (16 mmol) of 1,3,5-adamantanetricarboxylic acid and 8.99 g (24 mmol) of 3,3'-diaminobenzidinetetraisopropanoimine [N,N',N",N'"-tetraisopropylidene-3,4,3',4'-biphenyltetramine] prepared in Example 1 in 100 g of dioxane at room temperature in a nitrogen atmosphere. The coating composition was passed through a filter with a pore size of 0.1 micron and was applied to an 8-inch silicon wafer by spin coating. This was heated at 300°C in a nitrogen atmosphere for thirty minutes and was further heated at 400°C for thirty minutes to yield a film. The infrared absorption spectrum of the resulting polymer film was determined to find that the target crosslinked polybenzimidazole film was formed. The film has a film thickness of 360 nm.

### Example 18

A coating composition having a monomer concentration of 9.6 percent by weight was prepared by the procedure of Example 17, except for using 4.88 g (19.2 mmol) of 3,3'-diaminobenzidinemonoisopropanoimine [N'-isopropylidene-3,4,3',4'-biphenyltetramine] prepared according to Example 2 and 1.41 g (4.8 mmol) of 3,3'-diaminobenzidinediisopropanoimine [N',N'"-diisopropylidene-3,4,3',4'-biphenyltetramine] as aromatic polyamine derivatives instead of N,N',N",N'"-tetraisopropylidene-3,4,3',4'-biphenyltetramine. By using this coating composition, a polymer film was prepared by the procedure of Example 17, and the infrared absorption spectrum of the resulting polymer film was determined to find that the target crosslinked polybenzimidazole film was formed. The film has a film thickness of 300 nm.

### Comparative Example 3

The procedure of Example 17 was repeated, except for using 1,3,5-adamantanetricarboxylic acid, and 3,3'-diaminobenzidine instead of N,N',N",N'"-tetraisopropylidene-3,4,3',4'-biphenyltetramine in the same amounts as in Example 17, to find that the solubility in the solvent (dioxane) was low. Consequently, a coating composition having a monomer concentration of 2.3 percent by weight was prepared by the procedure of Example 17, except for using 1.07 g (4 mmol) of 1,3,5-adamantanetricarboxylic acid and 1.29 g (6 mmol) of 3,3'-diaminobenzidine. By using this coating composition, a polymer film was prepared by the procedure of Example 17, and the infrared absorption spectrum of the film was determined to find that the target crosslinked polybenzimidazole film was formed. The film has a film thickness of less than 20 nm.

### Example 19

A coating composition having a monomer concentration of 9.1 percent by weight was prepared by dissolving 3.76 g (14 mmol) of 1,3,5-adamantanetricarboxylic acid and 6.22 g (21 mmol) of 3,3'-dihydroxybenzidinediisopropanoimine [N,N'-diisopropylidene-3,3'-dihydroxybenzidine] prepared in Example 6 in 100 g of propylene glycol monomethyl ether (PGME) at room temperature in a nitrogen atmosphere. The coating composition was passed through a filter with a pore size of 0.1 micron and was applied to an 8-inch silicon wafer by spin coating. This was heated at 300°C in a nitrogen atmosphere for thirty minutes and was further heated at 400°C for thirty minutes to yield a film. The infrared absorption spectrum of the resulting polymer film was determined to find that a target crosslinked polybenzoxazole film was formed. The film has a film thickness of 300 nm.

### Comparative Example 4

The procedure of Example 19 was repeated, except for using 1,3,5-adamantanetricarboxylic acid, and 1.30 g (6 mmol) of 3,3'-dihydroxybenzidine instead of 3,3'-diacetoxybenzidinediacetylamide in the same amounts as in Example 19, to find that the solubility in the solvent (propylene glycol monomethyl ether: PGME) was low. Consequently, a coating composition having a monomer concentration of 2.3 percent by weight was prepared by the procedure of Example 19, except for using 1.07 g (4 mmol) of 1,3,5-adamantanetricarboxylic acid and 1.30 g (6 mmol) of 3,3'-dihydroxybenzidine. By using this coating composition, a polymer film was prepared by the procedure of Example 9, and the infrared absorption spectrum of the film was determined to find that a target crosslinked polybenzoxazole film was formed. The film has a film thickness of less than 20 nm.

### Comparative Example 5

In a flask equipped with a stirrer and a condenser were placed 5.37 g (20 mmol) of 1,3,5-adamantanetricarboxylic acid, 6.43 g (30 mmol) of 3,3'-diaminobenzidine, and 100 g of polyphosphoric acid, and the mixture was heated with stirring at 200°C in a nitrogen atmosphere for twelve hours. After cooling, the reaction mixture was combined with water, the precipitated solid was collected by filtration, was washed with an aqueous sodium hydrogen carbonate solution, water, and methanol and thereby yielded a polybenzimidazole as a solid. An attempt was made to dissolve the solid polybenzimidazole in N-methylpyrrolidone (NMP: solvent) but ended in fail. The polybenzimidazole could not be formed into a thin film by spin coating, and the target thin film was not obtained.

## Claims

1. An aromatic polyamine derivative represented by following Formula (1): 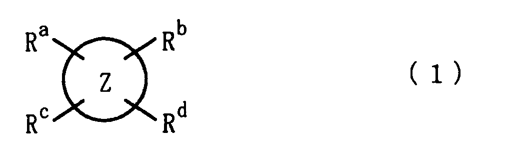 wherein Ring Z represents a monocyclic or polycyclic aromatic ring; R^{a}, R^{b}, R^{c}, and R^{d} are each a substituent bound to Ring Z, wherein R^{a}, R^{b} are the same as or different from each other and each represent a protected or unprotected amino group, and R^{c} and R^{d} are the same as or different from each other and each represent a protected or unprotected amino group, a protected or unprotected hydroxy group, or a protected or unprotected mercapto group, and wherein at least one of R^{a}, R^{b}, R^{c}, and R^{d} represents an amino group protected by an alkylidene group.
